(19) Europäisches Patentamt
European Patent Office
Office européen des brevets

(11) Publication number: **0 063 873**
**A1**

(12) # EUROPEAN PATENT APPLICATION

(21) Application number: **82301757.9**

(22) Date of filing: **02.04.82**

(51) Int. Cl.[3]: **C 07 C 65/24**, C 07 C 79/46, C 07 C 51/367, C 07 C 143/74, C 07 C 103/26, A 01 N 37/40, A 01 N 37/48, A 01 N 41/06

(30) Priority: **28.04.81 GB 8113122**

(43) Date of publication of application: **03.11.82 Bulletin 82/44**

(84) Designated Contracting States: **AT BE CH DE FR GB IT LI LU NL SE**

(71) Applicant: **IMPERIAL CHEMICAL INDUSTRIES PLC, Imperial Chemical House Millbank, London SW1P 3JF (GB)**

(72) Inventor: **Cartwright, David, 1 Stonehaven Drive, Woodley Reading Berkshire (GB)**
Inventor: **Collins, David John, 10 Ardwell Close, Crowthorne Berkshire (GB)**

(74) Representative: **Downes, John Edward et al, Imperial Chemical Industries PLC Legal Department: Patents Thames House North Millbank, London SW1P 4QG (GB)**

(54) **Haloalkoxy-substituted diphenyl ethers; their use as herbicides and in herbicidal compositions and processes of making them.**

(57) Compounds of the formula (I)

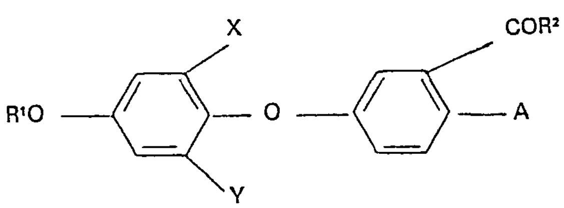

(I)

wherein $R^1$ is $C_1$-$C_2$ haloalkyl containing F or Cl, X and Y are H, halogen, alkyl, CN, $NO_2$ or $CF_3$; A is H, $CH_3$, $NO_2$, halogen, CN or $CF_3$; and $R^2$ is (a) -OH (b) -OM wherein M is a cation; (c) -$OR^3$ wherein $R^3$ is optionally substituted phenyl, alkyl, alkenyl or alkynyl (d) -$NR^4R^5$ wherein each group $R^4$ and $R^5$ is hydrogen or optionally substituted alkyl, alkenyl or phenyl or (e) -$NHSO_2R^3$ or -$NMSO_2R^3$. The compounds are useful as herbicides.

EP 0 063 873 A1

ACTORUM AG

COMPLETE DOCUMENT

HALOALKOXY- SUBSTITUTED DIPHENYL ETHERS; THEIR USE AS HERBICIDES AND IN HERBICIDAL COMPOSITIONS AND PROCESSES OF MAKING THEM

This invention relates to diphenyl ether compounds useful as herbicides, and to herbicidal compositions and processes utilising the compounds.

According to the present invention, there are provided diphenyl ether compounds of the formula (I):-

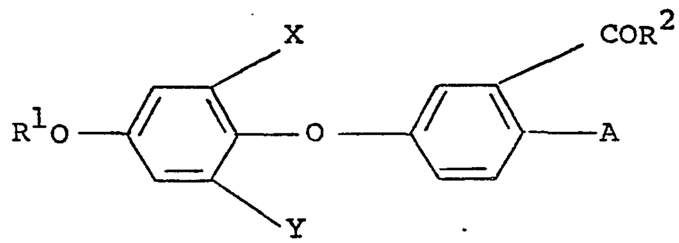

(I)

wherein $R^1$ is a haloalkyl group of 1 or 2 carbon atoms containing one or more fluorine or chlorine atoms; X and Y are each independently hydrogen, halogen, alkyl, cyano, nitro, or trifluoromethyl, A is hydrogen, nitro, halogen, $CH_3$, cyano, or trifluoromethyl; and $R^2$ is (a) -OH, (b) -OM wherein M is a cation, (c) $OR^3$ wherein $R^3$ is optionally substituted phenyl, alkyl, alkenyl or alkynyl (d) $-NR^4R^5$ wherein each of the groups $R^4$ and $R^5$ may be hydrogen or optionally substituted alkyl, alkenyl, alkynyl or phenyl, or (e) $-NHSO_2R^3$, or $-NMSO_2R^3$ wherein M is a cation.

The term halogen in the foregoing definition is intended to include fluorine, chlorine, bromine, and iodine. The haloalkyl group $R^1$ may be for example an alkyl group of one or two carbon atoms. The halogen part of the haloalkyl group may comprise one or more fluorine or chlorine atoms.

Examples of haloalkyl groups include $CF_3$-, $ClCF_2$, $CF_2H$-, $CH_3CF_2$ and $CHF_2CF_2$-.

When X or Y (or both) is an alkyl group, it may be for example an alkyl group of 1 to 4 carbon atoms, for example a methyl group.

When $R^2$ is a group -OM, the cation M may be a metal cation or an ammonium or substituted ammonium cation. Examples of metal cations include alkali metal cations for example sodium, potassium, lithium and alkaline earth metal cations, for example, calcium and magnesium.

Examples of substituted ammonium cations include mono-, di-, tri- and tetra- alkyl ammonium cations in which the one, two, three or four alkyl groups may each be of 1 to 6 carbon atoms and may optionally be substituted by one or more hydroxy or phenyl groups.

The group $R^3$ may for example be an alkyl group of 1 to 6 carbon atoms or an alkenyl or alkynyl group of 2 to 6 or more carbon atoms. Examples of substituents which may be present when $R^3$ is a substituted phenyl, alkyl, alkenyl or alkynyl group include halogen, trifluoromethyl, cyano, carboxy, -COOM, alkoxycarbonyl (e.g. alkoxycarbonyl containing from 2 to 5 carbon atoms), amino, mono- and di-alkylamino wherein the alkyl groups have for example 1 to 6 carbon atoms, alkylthio (e.g. alkylthio of 1 to 6 carbon atoms), alkoxy (e.g. alkoxy of from 1 to 6 carbon atoms) and phenyl.

When $R^4$ or $R^5$ is a substituted alkyl, alkenyl, alkynyl, or phenyl group, the substituents may have any of the values recited for the group $R^3$ above.

Within the class of compounds defined above, one subclass comprises compounds in which $R^1$ is a $ClCF_2$, $CHF_2$, or $CF_3$ group; X is hydrogen, chlorine, or $NO_2$; Y is hydrogen, fluorine or chlorine; $R^2$ is -OH, -OM, $-NH_2$, $-NHSO_2CH_3$, or $-NMSO_2CH_3$; and A is hydrogen, chlorine or $NO_2$.

Particular examples of compounds according to the invention include the compounds listed in Table 1 below :-

TABLE 1

| COMPOUND NO | $R^1$ | X | Y | $R^2$ | A | MELTING POINT |
|---|---|---|---|---|---|---|
| 1 | $CHF_2$- | H | H | OH | $NO_2$ | 131-133 |
| 2 | $CHF_2CF_2$- | H | H | $OCH_3$ | $NO_2$ | Oil |
| 3 | $CHF_2$- | Cl | H | OH | $NO_2$ | 101-103 |
| 4 | $CHF_2$- | Cl | H | $NHSO_2CH_3$ | $NO_2$ | 129-130 |
| 5 | $CHF_2$- | H | H | $NHSO_2CH_3$ | $NO_2$ | 141-142 |

Further examples of compounds falling within the scope of the invention are listed in Table 2 below:-

TABLE 2

| COMPOUND NUMBER | $R^1$ | X | Y | $R^2$ | A |
|---|---|---|---|---|---|
| 6 | $CHF_2$- | Cl | Cl | $NHSO_2CH_3$ | $NO_2$ |
| 7 | $CF_3$- | Cl | F | $NHSO_2CH_3$ | $NO_2$ |
| 8 | $CF_3$- | Cl | H | $OCH_3$ | $NO_2$ |
| 9 | $ClCF_2$- | H | H | $NHSO_2CH_3$ | $NO_2$ |
| 10 | $CF_3$- | Cl | F | $NHSO_2CH_3$ | Cl |
| 11 | $CF_3$- | Cl | F | OH | Cl |
| 12 | $ClCF_2$- | $NO_2$ | H | OH | Cl |
| 13 | $CF_3$- | Cl | H | OH | H |
| 14 | $CF_3$- | Cl | F | OH | H |
| 15 | $CHF_2$- | Cl | H | $NH_2$ | $NO_2$ |

The structural formula (I) given above is believed to be the one which best represents the structure of the compounds of the invention. For some compounds within the scope of the formula (I) it may be possible in principle for tautomeric forms of the compound to exist, in which a hydrogen atom may be transposed from one position to another within the molecule, the chemical bonds between the atoms of the molecule being consequently rearranged. The structural formula (I) is intended to represent and to include such tautomeric forms, insofar as they may exist. Where a compound falling within the scope of formula (I) is capable of existing in optically isomeric forms, the present invention includes the separate D and L forms of the compounds as well as mixtures of the D and L forms in all proportions. As normally prepared by chemical synthesis such optically isomeric compounds will be obtained as mixtures of equal proportions of the D and L forms (i.e. racemic mixtures).

The invention further provides processes for preparing compounds of formula (I) above. One such process is outlined in Scheme A below:-

Scheme A

(a)

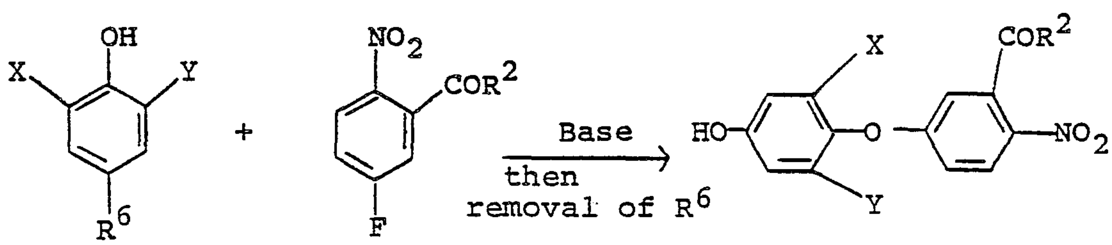


(II) (III)

(b) (III) + $R^1Cl$

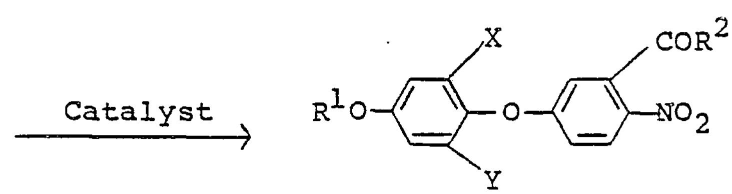


(IV)

According to Scheme A, a suitably substituted hydroquinone in which $R^6$ is H if X and Y are both hydrogen or a protecting group e.g. methyl if X or Y is not hydrogen is reacted with a 5-fluoro-2-nitrobenzoic derivative (II) in the presence of a base to give the hydroxy diphenyl ether derivative (III). The reaction is preferably carried out in a solvent or diluent which is inert towards the reactants; such solvents include liquid hydrocarbons, ketones, dimethylformamide, dimethylsulphoxide, and N-methylpyrrolidone. The reaction is preferably carried out with exclusion of atmospheric oxygen since hydroquinone is susceptible to oxidation.

The base used in the reaction may be, for example, an alkali metal carbonate (e.g. anhydrous $K_2CO_3$) or an alkaline earth metal hydroxide (e.g. $Ca(OH)_2$). The amount of base will depend on the nature of the group $R^2$. When this is an OH or $-NHSO_2R^3$ group, for example, two molar equivalents of base will be required. When $R^2$ is an OM group, for example, only one molar equivalent will be required.

A possible side reaction in stage (a) of the process when hydroquinone itself is used is the reaction of both of the hydroxy groups of the hydroquinone molecule with the 5-fluoro-2-nitrobenzoic derivative (II). The use of calcium hydroxide as a base tends to reduce this side reaction. When a substituted hydroquinone is used the protecting group $R^6$ is removed before stage (b).

In stage (b) of the process a metal salt of the hydroxydiphenyl ether (III) is reacted with an appropriate halogenoalkane to give a diphenyl ether (IV) according to the invention. The metal salt may be for example, a sodium or potassium salt, and may conveniently be prepared by dissolving the hydroxydiphenyl ether (III) in an aqueous solution containing an appropriate amount of sodium or potassium hydroxide. The reaction with the halogenoalkane may conveniently be carried out in a solvent comprising a mixture of water and an organic solvent, for example tetrahydrofuran. Preferably a phase-transfer catalyst is also

present in the reaction mixture, for example cetyl trimethyl ammonium chloride. The reaction is preferably accelerated by heating, for example to the reflux temperature of the mixture. The halogenoalkanes are highly volatile liquids and in order to retain them in the reaction flask it is necessary to use a condenser cooled with a mixture of solid carbon dioxide and a solvent (e.g. acetone). Alternatively, the reaction may be conducted in a sealed vessel. When using an open reaction vessel, reaction tends to be rather slow and may take several days to reach its maximum extent. The diphenyl ether derivative (IV) prepared as described above may be converted into other diphenyl ether derivatives according to the invention by standard chemical procedures. Thus, when (IV) is an ester, it may be hydrolysed to the corresponding carboxylic acid by, for example, treatment with a mineral acid. The carboxylic acid (IV, $R^2$=OH) may then be converted to the corresponding acid chloride which can be reacted with amines $R^4R^5NH$ to prepare amides, with sulphonamides $R^3SO_2NH_2$ to prepare N-alkane sulphonamides, or with other alcohols to prepare further esters of the carboxylic acid (IV, $R^2$=OH).

A further process for preparing compounds according to the invention is outlined in Scheme B.

Scheme B

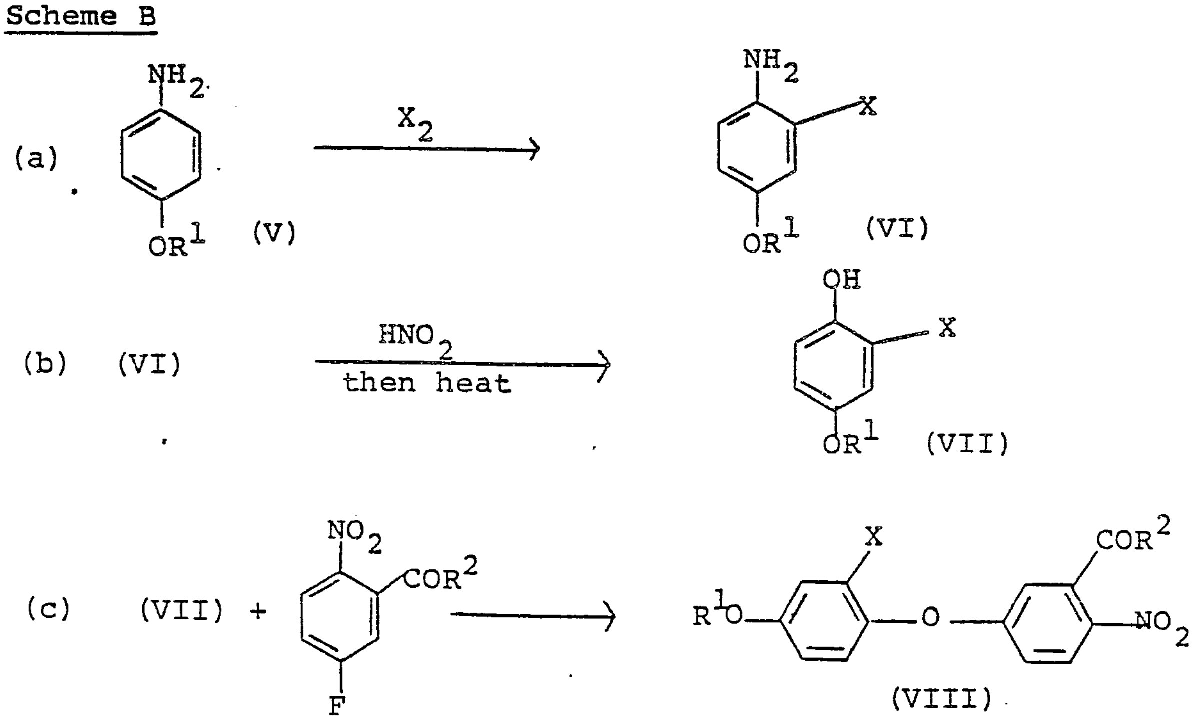

According to Scheme B, a p-amino phenyl ether (V) is treated in an inert solvent with a halogenating agent $X_2$. This may be for example elemental chlorine, bromine or iodine (but not elemental fluorine since this is not suitable as a fluorinating agent) or a halogen carrier, for example N-bromo- or N-chloro-succinimide. The X-substituted phenyl ether (V) so obtained is then treated in acid solution with nitrous acid to form the corresponding diazonium salt. This is then decomposed by heating to give the phenol (VI). In stage (c) the phenol (VI) is treated with a 5-fluoro-2-nitrobenzoic derivative in the presence of base to give the X-substituted diphenyl ether (VII).

The p-amino phenyl ether (V) required as starting material for stage (a) of Scheme B may be prepared by a variety of methods. Thus, for example, p-aminophenol may be reacted with a halide $R^1Cl$ in the presence of a base, or with a halogenoalkene (e.g. tetrafluoroethylene). In the case where $R^1$ is to be $CF_3$, the p-aminophenol may be reacted with a mixture of $CCl_4$ and HF.

A further process for preparing compounds according to the invention is outlined in Scheme C.

Scheme C

(a)

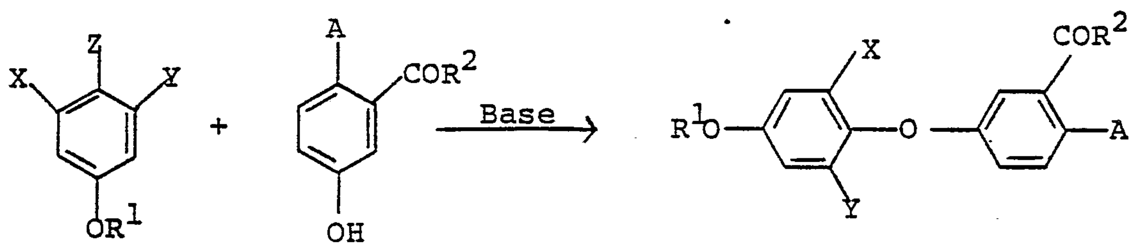


(IX)

According to Scheme C, an X,Y,Z-substituted phenyl ether (IX) wherein Z is chlorine or fluorine is reacted in presence of a base with a 3-hydroxybenzoic acid derivative to give the diphenyl ether (X).

By way of example, in the case when A is hydrogen and $R^2$ is OH, a typical procedure comprises first preparing the di-salt of 3-hydroxybenzoic acid by adding the stoichiometric amount of a methanol solution of sodium or potassium hydroxide to a methanol solution of 3-hydroxybenzoic acid and then removing the solvent. The remaining di-sodium or di-potassium salt is then taken up in an aprotic solvent, preferably a polar aprotic solvent (e.g. dimethyl formamide or dimethyl sulphoxide) and heated with the compound (IX). The reaction temperature is not critical; the reaction may for example be carried out at temperatures in the range from 100-180°C. The free acid (X) can be obtained at the end of the reaction by treating the reaction mixture with an excess of a strong mineral acid. One convenient way to isolate the acid (X) is to pour the cooled reaction mixture into water, extract the mixture with a water-immiscible organic solvent (e.g. chloroform or toluene) to remove unreacted starting material (IX) and the reaction solvent, and then acidify the aqueous solution to precipitate the acid (X).

The starting material (IX) may be prepared by reaction of a 3,4,5-X,Z,Y-substituted phenol with a halogenoalkane $R^1Cl$ or with a halogenoalkene in the same way as described above for the preparation of the p-amino phenyl ether (V).

Scheme C shows the substituent A as being present in the 3-hydroxybenzoic acid derivative which is reacted with the compound IX. However, if desired, when A is a substituent other than hydrogen, it may be introduced, by reaction of the diphenyl ether (X wherein A is hydrogen) with the appropriate electrophilic reagent.

0063873

Thus, where A is to be a nitro group, the diphenyl ether is treated with a nitrating agent, for example a mixture of nitric and sulphuric acids or potassium nitrate/sulphuric acid. Where A is to be chlorine, the diphenyl ether (X) may be dissolved in a solvent (e.g. glacial acetic acid) and treated for example with gaseous chlorine.

Examples of starting materials (II) for use in Scheme A include the following:-

Methyl 5-fluoro-2-nitrobenzoate

N-methanesulphonyl 5-fluoro-2-nitrobenzamide

Examples of starting materials (V) for use in Scheme B include the following:-

4-Amino (chlorodifluoromethoxy)benzene

4-Amino (trifluoromethoxy)benzene

4-Amino (difluoromethoxy)benzene

4-Amino (1,1,2,2-tetrafluoroethoxy)benzene

Examples of starting materials (IX) for use in Scheme C include the following:-

3,5-dichloro-4-fluoro(chlorodifluoromethoxy)benzene

3,5-dichloro-4-fluoro(trifluoromethoxy)benzene

3,5-dichloro-4-fluoro(difluoromethoxy)benzene

3-chloro-4-nitro(trifluoromethoxy)benzene

3-chloro-4-nitro(chlorodifluoromethoxy)benzene

The compounds of the invention are useful as herbicides. In another aspect, therefore, the invention provides a process of inhibiting the growth of unwanted plants, which comprises applying to the plants, or to the locus thereof, a phytotoxic amount of a compound of the formula (I) as hereinbefore defined. The amount of the compound to be applied in the process may vary, depending upon the particular compound chosen, and the identity of the plant species whose growth is to be inhibited, but in general amounts from 0.5 to 10 kilograms per hectare will be suitable; in many cases from 3 to 5 kilograms per hectare will be appropriate. The skilled worker in the herbicide art will readily be able to establish appropriate application rates by standard procedures without undue experimentation.

The compounds of the invention are effective in controlling a variety of unwanted plants; in particular, they may be used to control species of the plant genus Cyperus, which many existing herbicides fail to control. The compounds may be applied to the above-ground parts of unwanted plants (post-emergence application) or they may be applied to the soil to prevent the growth of plants from seeds present in the soil (pre-emergence application). In general the compounds are more effective when used as pre-emergence treatments.

The compounds used in the process of the invention are preferably applied in the form of a composition, in which the active ingredient is mixed with a carrier comprising a solid or liquid diluent. In another aspect, therefore, the invention provides a herbicidal composition, comprising as an active ingredient a compound of the formula (I) as hereinbefore defined, in admixture with a solid or liquid diluent. Preferably the composition also comprises a surface-active agent.

The solid compositions of the invention may be for example, in the form of dusting powders, or may take the form of granules. Suitable solid diluents include, for example, kaolin, bentonite, kieselguhr, dolomite, calcium carbonate, talc, powdered magnesia, and Fuller's earth.

Solid compositions may also be in the form of dispersible powders or grains comprising in addition to the active ingredient, a wetting agent to facilitate the dispersion of the powder or grains in liquids. Such powders or grains may include fillers, suspending agents and the like.

Liquid compositions include aqueous solutions, dispersions and emulsions containing the active ingredient preferably in the presence of one or more surface active agents. Water or organic liquids may be used to prepare solutions, dispersions, or emulsions of the active ingredient. The liquid compositions of the invention may also contain one or more corrosion inhibitors for example lauryl isoquinolinium bromide.

Surface active agents may be of the cationic, anionic or non-ionic type. Suitable agents of the cationic type include for example quaternary ammonium compounds, for example cetyltrimethyl ammonium bromide. Suitable agents of the anionic type include for example soaps, salts of aliphatic mono-esters of sulphuric acid, for example sodium lauryl sulphate; and salts of sulphonated aromatic compounds, for example dodecyl-benzenesulphonate, sodium, calcium and ammonium ligno-sulphonate, butylnaphthalene sulphonate, and a mixture of the sodium salts of diisopropyl- and triisopropyl-naph-thalenesulphonic acid. Suitable agents of the non-ionic type include, for example, the condensation products of ethylene oxide with fatty alcohols such as oleyl alcohol and cetyl alcohol, or with alkyl phenols such as octylphenol, nonylphenol, and octylcresol. Other non-ionic agents are the partial esters derived from long chain fatty acids and hexitol anhydrides, for example sorbitol monolaurate; the condensation products of the said partial esters with ethylene oxide and the lecithins.

The compositions which are to be used in the form of aqueous solutions, dispersions or emulsions are generally supplied in the form of concentrate containing a high proportion of the active ingredient, the concentrate being diluted with water before use. These concentrates are usually required to withstand storage for prolonged periods and after such storage to be capable of dilution with water in order to form aqueous preparations which remain homogeneous for a sufficient time to enable them to be applied by conventional spray equipment.

The compositions of the invention may contain, in addition to carriers and surface-active agents, various other constituents to increase their usefulness. They may contain, for example, buffering salts to maintain the pH of the composition within a desired range; antifreeze agents, for example urea or propylene glycol; adjuvants, for example, oils and humectants; and sequestrants, for example

citric acid and ethylenediaminetetracetic acid, which help to prevent the formation of insoluble precipitates when the compositions are diluted with hard water. Aqueous dispersions may contain anti-settling agents and anti-caking agents.

The compositions may in general contain a dye or pigment to impart a characteristic colour. Agents for increasing viscosity may be added to reduce the formation of fine droplets during spraying, and thereby reduce spray drift. Other additives useful for particular purposes will be known to those skilled in the formulation art.

In general concentrates may conveniently contain from 10 to 85% and preferably from 25 to 60% by weight of active ingredient. Dilute preparations ready for use may contain varying amounts of the active ingredient, depending upon the purpose for which they are to be used; however, dilute preparations suitable for many uses contain between 0.01% and 10% and preferably between 0.1% and 1% by weight of the active ingredient.

The compounds of the invention can be used in association (for example in the form of a mixture) with another herbicide.

Examples of such herbicides are:

A. benzo-2,1,3-thiadiazin-4-one-2,2-dioxides such as 3-isopropylbenzo-2,1,3-thiadiazin-4-one-2,2-dioxide (bentazon);

B. hormone herbicides, particularly the phenoxy alkanoic acids such as 4-chloro-2-methylphenoxy acetic acid (MCPA), 2-(2,4-dichlorophenoxy)propionic acid (dichlorprop), 2,4,5-trichlorophenoxyacetic acid (2,4,5-T), 4-(4-chloro-2-methylphenoxy)butyric acid (MCPB), 2,4-dichlorophenoxyacetic acid (2,4-D), 4-(2,4-dichlorophenoxy)butyric acid (2,4-DB), 2-(4-chloro-2-methylphenoxy)propionic acid (mecoprop), and their derivatives (e.g. salts, esters and amides);

C. 3-[4-(4-halophenoxy)phenyl]-1,1-dialkylureas such as 3-[4-(4-chlorophenoxy)phenyl]-1,1-dimethylurea (chloroxuron);

D. dinitrophenols and their derivatives (e.g. acetates) such as 2-methyl-4,6-dinitrophenol (DNOC), 2-t-butyl-4,6-dinitrophenol (dinoterb), 2-secbutyl-4,6-dinitrophenol (dinoseb) and its ester, dinoseb acetate;

E. dinitroaniline herbicides such as N',N'-diethyl-2,6-dinitro-4-trifluoromethyl-m-phenylenediamine (dinitramine), 2,6-dinitro-N,N-dipropyl-4-trifluoromethylaniline (trifluralin) and 4-methysulphonyl-2,6-dinitro-N,N-dipropylaniline (nitralin);

F. phenylurea herbicides such as N'-(3,4-dichlorophenyl)-N,N-dimethylurea (diuron) and N,N-dimethyl-N'-[3-(trifluoromethyl)phenyl]urea (flumeturon);

G. phenylcarbamoyloxyphenylcarbamates such as 3-[methoxycarbonylamino]phenyl (3-methylphenyl)-carbamate (phenmedipham) and 3-[ethoxycarbonylamino]phenyl phenylcarbamate (desmedipham);

H. 2-phenylpyridazin-3-ones such as 5-amino-4-chloro-2-phenylpyridazin-3-one (pyrazon);

I. uracil herbicides such as 3-cyclohexyl-5,6-trimethyleneuracil (lenacil), 5-bromo-3-sec-butyl-6-methyluracil (bromacil) and 3-t-butyl-5-chloro-6-methyluracil terbacil);

J. triazine herbicides such as 2-chloro-4-ethylamino-6-(i-propylamino)-1,3,5-triazine (atrazine), 2-chloro-4,6-di (ethylamino)-1,3,5-triazine (simazine) and 2-azido-4-(i-propylamino)-6-methylthio-1,3,5-triazine (aziprotryne);

K. 1-alkoxy-1-alkyl-3-phenylurea herbicides such as 3-(3,4-dichlorophenyl)-1-methoxy-1-methylurea (linuron), 3-(4-chlorophenyl)-1-methoxy-1-methylurea (monolinuron) and 3-(4-bromo-4-chlorophenyl)-1-methoxy-1-methylurea (chlorobromuron).

L. thiolcarbamate herbicides such as S-propyl dipropylthiocarbamate (verolate);

M. 1,2,4-triazin-5-one herbicides such as 4-amino-4,5-dihydro-3-methyl-6-phenyl-1,2,4-triazine-5-one (metamitron) and 4-amino-6-t-butyl-4,5-dihydro-3-methylthio-1,3,4-triazin-5-one (metribuzin);

N. benzoic acid herbicides such as 2,3,6-trichlorobenzoic acid (2,3,6-TBA), 3,6-dichloro-2-methoxybenzoic acid (dicamba) and 3-amino-2,5-dichlorobenzoic acid (chloramben);

O. anilide herbicides such as N-butoxymethyl-α-chloro-2',6'-diethylacetanilide (butachlor), the corresponding N-methoxy compound (alachlor), the corresponding N-i-propyl compound (propachlor) and 3',4'-dichloropropionanilide (propanil);

P. dihalobenzonitrile herbicides such as 2,6-dichlorobenzonitrile (dichlobenil), 3,5-dibromo-4-hydroxybenzonitrile (bromoxynil) and 3,5-diiodo-4-hydroxybenzonitrile (ioxynil).

Q. haloalkanoic herbicides such as 2,2-dichloropropionic acid (dalapon), trichloroacetic acid (TCA) and salts thereof;

R. diphenylether herbicides such as 4-nitrophenyl 2-nitro-4-trifluoromethylphenyl ether (fluorodifen), methyl 5-(2,4-dichlorophenoxy)-2-nitrobenzoate (bifenox), 2-nitro-5-(2-chloro-4-trifluoromethylphenoxy)benzoic acid, 2-chloro-4-trifluoromethylphenyl 3-ethoxy-4-nitrophenyl ether and the compounds of European Patent Specification Publication No. 3416 (the disclosure of which Specification is incorporated herein by reference); and

S. miscellaneous herbicides including N,N-dimethyl-diphenylacetamide (diphenamid), N-(1-naphthyl)-phthalamic acid (naptalam) and 3-amino-1,2,4-triazole.

T. bipyridylium herbicides such as those in which the active entity is the 1,1'-dimethyl-4,4'-dipyridylium ion (paraquat) and those in which the active entity is the 1,1'-ethylene-2,2'dipyridylium ion (diquat).

U. Aryloxyphenoxypropionic acids and their derivatives (salts, esters, amides, and the like).

Examples of such acids are:

2-[4-(5-trifluoromethylpyridyl-2-oxy)phenoxy]propionic acid.
2-[4-(4-trifluoromethylphenoxy)phenoxy]propionic acid.
2-[4-(2,4-dichlorophenoxy)phenoxy]propionic acid.
2-[4-(5- or 6-chlorobenzoxazolyl-2-oxy)phenoxy]-propionic acid
4-methyl-4-(4-trifluoromethylphenoxy)phenoxybut-2-enoic acid.

The invention is illustrated by the following Examples, in which unless otherwise stated all parts are by weight and all temperatures in degrees Centigrade.

EXAMPLE 1

This Example illustrates the preparation of 5-(4-difluoromethoxyphenoxy)-2-nitrobenzoic acid.

(a) Methyl 5-fluoro-2-nitrobenzoate (6 g), dissolved in dimethylsulphoxide (120 ml) was stirred for 4.5 hours at 95°C under an atmosphere of argon with anhydrous calcium hydroxide (25 g) and hydroquinone (3.37 g). The mixture was then cooled and agitated with dilute hydrochloric acid and ether. The ether layer was separated from the acidic aqueous layer and washed once with dilute hydrochloric acid and twice with water. The ether solution was then dried ($MgSO_4$) and evaporated to give an oil. Trituration with hexane gave a solid (7.5 g) which was recrystallised from ethyl acetate/hexane to give the methyl ester of 5-(4-hydroxyphenoxy)-2-nitrobenzoic acid. This was hydrolysed to 5-(4-hydroxyphenoxy)-2-nitrobenzoic acid by treatment with hydrochloric acid.

(b) The acid (2.5 g) obtained as described in (a) was dissolved in tetrahydrofuran (30 ml) and added to aqueous sodium hydroxide solution (30 ml containing 1 molar equivalent). Cetyl triethylammonium chloride (50% aqueous solution, 0.5 ml) was added and the mixture stirred vigorously while gaseous chlorodifluoromethane was passed into the reaction flask. The chlorodifluoromethane was retained in the reaction flask by means of a condenser cooled with solid carbon dioxide. The mixture was heated under reflux for four days, with occasional additions of chlorodifluoromethane to make up for losses. The mixture was then agitated with ether and dilute hydrochloric acid. The ether layer was washed with water, dried ($MgSO_4$) and evaporated to give a brown oil (2.3 g). A one gram portion of the oil was purified by thin layer chromatography (2 mm thickness silica gel) using a mixture of chloroform, acetone, and acetic acid (80:20:10) as the solvent.

The 5-(4-difluoromethoxyphenoxy)-2-nitrobenzoic acid so obtained had a melting point of 131-133°C. The nuclear magnetic resonance spectrum and elemental analysis of this product were consistent with the structure assigned.

EXAMPLE 2

This Example illustrates a preparation of methyl 5-[4-(1,1,2,2-tetrafluoroethoxy)phenoxy]-2-nitrobenzoate (Compound no.2 of Table 1.

(a) Preparation of 4-amino (1,1,2,2-tetrafluoroethoxy)-benzene.

4-Nitro-(1,1,2,2-tetrafluoroethoxy)benzene (10 g) in *iso*-propanol (160 ml) and water (40 ml) was treated with iron powder (20 g) and 20 drops of concentrated hydrochloric acid. The reaction mixture was stirred and heated to reflux for 90 minutes. The mixture was filtered hot and the residue washed with *iso*propanol. The filtrate was evaporated to give a yellow oil. This was dissolved in ether and the ether solution dried ($MgSO_4$) and evaporated to give a yellow oil (8.9 g) identified as the required amine.

(b) Preparation of 4-hydroxy (1,1,2,2-tetrafluoroethoxy) benzene.

4-Amino-(1,1,2,2-tetrafluoroethoxy)benzene (8 g) was added to concentrated hydrochloric acid (35 ml) cooled to $0^{o}C$. The suspension which resulted was stirred vigorously while a solution of sodium nitrite (2.9 g) in water (5 ml) was added dropwise over a period of ten minutes. The mixture was stirred for a further 30 minutes to complete the reaction, and then filtered at $0-5^{o}$. The filtrate was poured into a solution of sodium fluoborate (9.5 g) in water (11 ml). The colourless solid which

separated was washed with dilute sodium fluoborate solution, then with ethanol and ether, and dried to give the diazonium fluoborate salt (7.9 g).

A sample (5.9 g) of the fluoborate so prepared was heated with stirring to 115-120° in acetic anhydride (30 ml) for 2.25 hours. The mixture was then allowed to cool and the excess of acetic anhydride removed under reduced pressure. The remaining oil was dissolved in isopropanol (50 ml) and made alkaline with 2-molar sodium hydroxide. The mixture was kept at room temperature for 4.5 hours, diluted with water and acidified with concentrated hydrochloric acid. The mixture was extracted with ether (2 x 200 ml). The extract was washed with aqueous sodium bicarbonate solution and then dried ($MgSO_4$) and evaporated to give a brown oil (4.1 g). The oil was distilled and the fraction boiling at 116-120°/22 Torr was collected; this rapidly crystallised to give the required phenol (1.8 g) with a melting point of 44-45°C.

(c) Preparation of compound no.2.

A portion (0.42 g) of the product from paragraph (b) was stirred and heated to reflux for 11 hours with methyl 5-fluoro-2-nitrobenzoate (0.4 g) and anhydrous potassium carbonate (0.5 g) in dry methyl ethyl ketone (5 ml). The mixture was filtered and the residue washed with methyl ethyl ketone.

The filtrate was evaporated to give a brown oil, which was purified by thin layer chromatography using a 2 mm layer of silica gel as the solid phase and a mixture of ether and petroleum ether (1:3) as the eluent. The colourless oil (0.34 g) so obtained was identified as compound no.2 of Table 1.

EXAMPLE 3

This Example illustrates a preparation of N-methanesulphonyl-5(2-chloro-4-difluoromethoxyphenoxy)2-nitrobenzamide (Compound no.4 of Table 1).

(a) Preparation of methyl 5-(2-chloro-4-methoxyphenoxy)-2-nitrobenzoate.

2-Chloro-4-methoxyphenol (6.23 g) and methyl 5-fluoro-2-nitrobenzoate (7.83 g) were stirred and heated to 80°C with anhydrous potassium carbonate (30 g) in dimethylsulphoxide (25 ml) for 3 hours. The mixture was then poured into water, acidified with dilute hydrochloric acid, and extracted with ethyl acetate. The extract was dried ($MgSO_4$) and evaporated to give an oil (13.8 g) which crystallised on standing and was identified as the required ester.

(b) Preparation of 5(2-chloro-4-hydroxyphenoxy)-2-nitrobenzoic acid.

The product (11.5 g) from paragraph (a) was fused with pyridine hydrochloride (35 g) at 170°C for 30 minutes, cooled to 100°C, and poured into cold water. The mixture was extracted with ethyl acetate. The extract was dried and evaporated to give a red oil. This crystallised slowly, and was triturated with hexane to give a solid (7.53 g). This was found to be a mixture in which the principal component was 5(2-chloro-4-methoxy)-2-nitrobenzoic acid. A sample of this solid (2.2 g) was fused with pyridine hydrochloride at 195-200°C for a further 30 minutes, cooled, and poured into dilute hydrochloric acid. The mixture was extracted with ethyl acetate extract was washed with dilute hydrochloric acid and then water. The extract was then dried ($MgSO_4$) treated with charcoal, and evaporated to give a brown oil (1.70 g). This was identified by its proton magnetic resonance spectrum as 5-(2-chloro-4-hydroxyphenoxy)-2-nitrobenzoic acid together with a proportion of the corresponding methyl ester.

(c) Preparation of 5(2-chloro-4-difluoromethoxyphenoxy)-2-nitrobenzoic acid (compound no.3 of Table 1).

The product (1.1 g) from paragraph (b) was dissolved in a solution of potassium carbonate (5 g) in water (50 ml). Toluene (50 ml) and benzyltriethylammonium chloride (2 g) and chlorodifluoromethane (excess) were added and the mixture stirred vigorously at 75° under a reflux condenser cooled by solid carbon dioxide in acetone. The mixture was heated under reflux for 7.5 hours each day for four consecutive days. The mixture was then shaken with ethyl acetate and dilute hydrochloric acid and the ethyl acetate layer separated, washed with water, dried ($MgSO_4$) and evaporated to give a brown oil. This was purified by thin layer chromatography using a layer of silica gel (2 mm) as the solid phase and a mixture of chloroform and acetic acid (90:10) as the eluent. Elution of the major component gave the required acid (0.51 g) with a melting point of 101-103°C.

(d) Preparation of compound 4.

The product (0.4 g) from paragraph (c) was heated under reflux in thionyl chloride (10 ml) for 1.5 hours. The excess of thionyl chloride was removed under reduced pressure and the remaining oil was taken up in methyl isobutyl ketone (20 ml) and heated and stirred under reflux for 2 hours with anhydrous potassium carbonate (2.5 g) and methanesulphonamide (0.15 g). The mixture was then cooled shaken with ethyl acetate and dilute hydrochloric acid. The ethyl acetate layer was separated, dried ($MgSO_4$) and evaporated to yield a buff oil, which crystallised on standing. This was recrystallised from a mixture of ether and hexane to give compound no.4 of Table 1, with a melting point of 129-130°C.

EXAMPLE 4

This Example illustrates a preparation of N-methanesulphonyl -5-(4-difluoromethoxyphenoxy)-2-nitrobenzamide (compound no.5 of Table 1).

5(4-Difluoromethoxyphenoxy)-2-nitrobenzoic acid (0.4 g, prepared as described in Example 1) was heated under reflux in thionyl chloride (10 ml) for 90 minutes. The excess of thionyl chloride was then removed under reduced pressure and the remaining oil taken up in dry methyl isobutyl ketone (15 ml). Anhydrous potassium carbonate (2 g) and methanesulphonamide (0.15 g) were then added and the mixture stirred and heated under reflux for 1 hour. The mixture was cooled and shaken with ethyl acetate and dilute hydrochloric acid. The ethyl acetate layer was separated, dried ($MgSO_4$) and evaporated to give a light brown oil. On trituration with hexane this solidified. Recrystallisation from a mixture of ethyl acetate and hexane gave compound no.5 as a white crystalline solid (0.28 g) with a melting point of 141-142°C.

EXAMPLE 5

This Example illustrates a preparation of N-methanesulphonyl 5-fluoro-2-nitrobenzamide, useful as an intermediate for preparing compounds of the invention by the process of Scheme A.

A solution of 5-fluoro-2-nitrobenzoic acid (9.0 g) in toluene (30 ml) was heated with thionyl chloride (3.8 ml) for 2 hours at 70°C. The solution was cooled and added dropwise to a solution of methanesulphonamide (4.62 g) in pyridine (15 ml) with ice cooling. The solution was stirred at room temperature for 2 hours and then poured into water, acidified, and extracted with ethyl acetate. The extract was washed with water, dried, and evaporated to give a solid. This was recrystallised from a mixture of ethyl acetate and petroleum to give the required amide (5.36 g) as off-white needles with a melting point of 178°C.

EXAMPLE 6

This Example illustrates a preparation of 3,5-dichloro-4-fluoro (chlorodifluoromethoxy)benzene, useful as a starting material (IX) for the preparation of compounds of the invention by the process of Scheme C.

(a) Preparation of 4-amino-3,5-dichloro-(chlorodifluoromethoxy)benzene.

4-Amino (chlorodifluoromethoxy)benzene (1 g) (containing 10% of 4-amino (trifluoromethoxy)benzene) in glacial acetic acid was cooled in a cold water bath while chlorine was passed in. A beige precipitate was collected and triturated with ether. The ether solution, upon evaporation, gave a brown oil (0.5 g) identified as the required dichloro compound. The insoluble residue from the trituration with ether was identified as the hydrochloride of the starting material; the starting material could be recovered by treatment with sodium hydroxide solution and extraction with ether, and then recycled for chlorination in acetic acid as described above.

(b) Preparation of 3,5-dichloro-4-fluoro (chlorodifluoromethoxy)benzene.

The dichloro compound (6.6 g) prepared as described in paragraph (a) was added dropwise to concentrated hydrochloric acid (30 ml) cooled in ice and water with stirring. The suspension so prepared was stirred and treated dropwise with a solution of sodium nitrite (4 g) in water (10 ml) while the temperature was maintained at 3°C by cooling. The solution was filtered and the filtrate added to sodium fluoborate (4 g) in the minimum of ice-cold water. The white precipitate was washed with cold water and dried to give the fluoborate salt (4.5 g). This was mixed with sand and heated in a bulb tube at 200°C. An orange oil distilled and was collected (0.5 g)

and identified as the required 3,5-dichloro-4-fluoro-(chlorodifluoromethoxy)benzene containing as an impurity about 10% of 3,5-dichloro-4-fluoro-(trifluoromethoxy)-benzene.

EXAMPLE 7

This Example illustrates a preparation of 3-chloro-4-nitro-(chlorodifluoromethoxy)benzene from 4-amino (chloro-difluoromethoxy)benzene.

(a) A 50:50 mixture of 4-amino (chlorodifluoromethoxy)-benzene and 4-amino(trifluoromethoxy)benzene (5 g) was added slowly with stirring to concentrated sulphuric acid (30 ml) cooled in ice. Concentrated nitric acid (2 ml) was then added with stirring and ice cooling. After 2 hours the mixture was allowed to warm to room temperature and then poured onto ice. The brown solid was taken up in ether and the ether solution washed with water and with sodium carbonate solution. Evaporation of the ether gave a dark solid (4 g) identified as a 50:50 mixture of 4-amino-3-nitro-(chlorodifluoromethoxy)benzene and 4-amino-3-nitro(trifluoromethoxy)benzene.

(b) A portion (3 g) of the product from paragraph (a) was added slowly to concentrated hydrochloric acid (30 ml) cooled on ice. The mixture was stirred and kept at 3°C while sodium nitrite (3 g) in water (15 ml) was added dropwise. The mixture was then stirred at 3°C for a further 45 minutes and filtered. The filtrate was poured slowly into a solution of cuprous chloride (3 g) in concentrated hydrochloric acid (30 ml). The mixture was allowed to warm to room temperature over a period of 1 hour and then poured onto ice. When the ice had melted, the mixture was extracted with ether. The ether extract was washed with water and brine and then dried and evaporated to give an orange oil identified as a mixture of 3-chloro-4-nitro-(chlorodifluoromethoxy)benzene and 3-chloro-4-nitro-(trifluoromethoxy)benzene.

Although this preparation was carried out using a 50:50 mixture of trifluoromethoxy- and chlorodifluoromethoxy starting materials, it is clear that the presence of either starting material does not affect the conversion of the other, and the preparation could be carried out with the starting materials mixed in any proportion, or with pure 4-amino (chlorodifluoromethoxy)benzene or 4-amino (trifluoromethoxybenzene).

## EXAMPLE 8

This Example illustrates a preparation of 4-hydroxy (chlorodifluoromethoxy)benzene, useful as a starting material for the preparation of compounds of the invention.

(a) A 50:50 mixture of 4-amino (chlorodifluoromethoxy)-benzene and 4-amino (trifluoromethoxy)benzene (5.5 g) was dissolved in concentrated hydrochloric acid (25 ml), cooled to -3°C and stirred while sodium nitrite (4 g) in water (10 ml) was added dropwise. The mixture was filtered and the orange filtrate added slowly to a solution of sodium fluoborate (4 g) in water (50 ml). The precipitated fluoborate salt was collected (1.1 g) and dried in air.

(b) The fluoborate salt (1.1 g) from paragraph (a) was added to cold acetic anhydride (25 ml) and the suspension stirred and heated to 130°C for 3 hours. The mixture was then left overnight. The excess of acetic anhydride was removed under reduced pressure and the residue triturated with ether. The ether solution was filtered and evaporated to give a yellow oil (0.6 g) identified as a 50:50 mixture of 4-acetoxy-(chlorodifluoromethoxy)-benzene and 4-acetoxy-(trifluoromethoxy)benzene.

(c) The acetoxy compounds (0.25 g) prepared in paragraph (b) were taken up in glacial acetic acid (1.5 ml) and heated with 48% hydrobromic acid (1 ml) for 2 hours at 100°C. The mixture was cooled and poured into water, and extracted with ether. The ether extract was washed with water and brine, dried, and evaporated to give a yellow oil. This was taken up again in ether and washed with sodium bicarbonate solution. Evaporation of the ether solution gave a yellow oil, identified as a 50:50 mixture of 4-hydroxy (chlorodifluoromethoxy)benzene and 4-hydroxy (trifluoromethoxy)benzene by mass spectrography.

Although this preparation was carried out using a 50:50 mixture of trifluoromethoxy- and chlorodifluoro-methoxy- starting materials, it is clear that the presence of either starting material does not affect the conversion of the other and the preparation could be carried out with the starting materials mixed in any proportion, or with pure 4-amino (chlorodifluoromethoxy)benzene or 4-amino (trifluoromethoxy)benzene.

## EXAMPLE 9

This Example illustrates the herbicidal properties of compounds 1 and 5 of Table 1. The compounds were formulated for test by mixing an appropriate amount with 0.7 ml of a solution of a mixture of "Tween" 85 and Synperonic NPE 1800 in cyclohexanone. "Tween" 85 is a Trade Mark for condensate of sorbitan trioleate with 20 molar proportions of ethylene oxide. Synperonic NPE 1800 is a Trade Mark for a condensate of p-nonylphenol with propylene oxide and ethylene oxide. The concentration of the "Tween"/"Synperonic" mixture was 5 grams per 100 ml of cyclohexanone and the ratio of "Tween" to "Synperonic" in the mixture was 1:2. The mixture of the compound and the cyclohexanone solution was shaken with glass beads and diluted to 7 ml with water.

0063873

The spray composition so prepared was sprayed on to young pot plants (post-emergence test) of the species named in Table 3 below, at a rate equivalent to 1000 litres per hectare. Damage to plants was assessed 14 days after spraying by comparison with untreated plants, on a scale of 0 to 3 where 0 is no effect and 3 represents 75 to 100% kill. In a test for pre-emergence herbicidal activity, seeds of the test species were placed on the surface of plastic trays of soil and were sprayed with the compositions prepared again as above, at the rate of 1000 litres per hectare. The seeds were then covered with further soil. Three weeks after spraying, the seedlings in the sprayed plastic trays were compared with the seedlings in unsprayed control trays, the damage being assessed on the same scale of 0 to 3. The results given in Table 3 below:-

TABLE 3

| Compound Number | Application rate (kg/ha) | Pre- or Post Emergence Application | Test Plants | | | | | |
|---|---|---|---|---|---|---|---|---|
| | | | Lt | To | Av | St | Ll | Cn |
| 1 | 10 | Pre | 3 | 2 | 1 | 3 | 0 | 3 |
| | | Post | 2 | 2 | 1 | 3 | 0 | 0 |
| 5 | 5 | Pre (a) | 3 | 3 | 2 | 3 | 2 | 2 |
| | | Post (b) | 3 | 2 | 0 | 3 | 0 | 0 |

(a) Damage assessed 15 days after treatment
(b) Damage assessed 13 days after treatment

The names of the test plants in Table 3 are as follows:-

| | |
|---|---|
| Lt | Lettuce |
| To | Tomato |
| Av | *Avena fatua* |
| St | *Setaria viridis* |
| Ll | Perennial rye-grass (*Lolium perenne*) |
| Cn | *Cyperus rotundus* |

EXAMPLE 10

This Example illustrates the herbicidal properties of compounds of Table 1. The compounds were submitted to herbicide tests as described below.

Each compound was formulated for test by mixing an appropriate amount of it with 1.8 ml of a solution containing 10 grams per 100 ml of a mixture of equal parts by weight of Span 80 and Tween 20 in methylcyclohexanone. Span 80 is a Trade Mark for a surface-active agent comprising sorbitan monolaurate. Tween 20 is a Trade Mark for a surface-active agent comprising a condensate of 20 molar proportions of ethylene oxide with sorbitan monolaurate. The mixture of the compound and the methylcyclohexanone solution was then shaken with glass beads and diluted to 45 ml with water. The spray compositions so prepared was sprayed on to young pot plants (post-emergence test) of the species named in the Table below, at a rate equivalent to 1000 litres per hectare. Damage to plants was assessed 13 days after spraying by comparison with untreated plants, on a scale of 0 to 5 where 0 is 0 to 20% damage and 5 is complete kill. In the Table of results, a dash (-) means that no test was made.

A test was also carried out to detect pre-emergence herbicidal activity. Seeds of the test species were placed on the surface of plastic trays of soil and were sprayed with the compositions at the rate of 1000 litres per hectare. The seeds were then covered with further soil. Seventeen days after spraying, the seedlings in the sprayed plastic trays were compared with the seedlings in unsprayed control trays, the damage being assessed on the same scale of 0 to 5.

The results of the tests are given in Table 4 below.

TABLE 4

| COMPOUND NUMBER | RATE OF APPLICATION kg/ha | PRE- OR POST-EMERGENCE APPLICATION | TEST PLANTS | | | | | | | | | | | | | | | | | | | | | | | |
|---|---|---|---|---|---|---|---|---|---|---|---|---|---|---|---|---|---|---|---|---|---|---|---|---|---|---|
| | | | Sb | Rp | Ct | Sy | Mz | Ww | Rc | Sn | Ip | Am | Pi | Ca | Ga | Xa | Ab | Co | Av | Dg | Al | St | Ec | Sh | Ag | Cn |
| 1 | 3 | Pre | 3 | 4 | 1 | 4 | 2 | 2 | 2 | 2 | 0 | 4 | 0 | - | 2 | 0 | 4 | 1 | 2 | 2 | 1 | 4 | 0 | 4 | 0 | 1 |
| 3 | 0.2 | Pre | 3 | 4 | 0 | 1 | 0 | 0 | 1 | 3 | 2 | 3 | 1 | - | 0 | 0 | 3 | 1 | 0 | 1 | 0 | 0 | 0 | 3 | 3 | 0 |
| | | Post | 3 | 4 | 1 | 1 | 2 | 1 | 1 | 3 | 2 | 3 | 1 | 4 | 1 | 2 | 1 | 1 | 0 | 2 | 0 | 3 | 1 | 3 | 0 | 0 |
| 4 | 0.2 | Pre | 4 | 5 | 0 | 0 | 2 | 1 | 1 | 4 | 2 | 4 | 1 | - | 2 | 3 | 4 | 4 | 1 | 3 | 1 | 2 | 1 | 4 | 0 | 0 |
| | | Post | 3 | 5 | 1 | 1 | 2 | 1 | 1 | 4 | 3 | 3 | 3 | 5 | 3 | 3 | 3 | 3 | 0 | 1 | 0 | 5 | 1 | 3 | 0 | 0 |

Names of test plants

| | |
|---|---|
| Sb | Sugar beet |
| Rp | Rape |
| Ct | Cotton |
| Sy | Soya Bean |
| Mz | Maize |
| Ww | Winter Wheat |
| Rc | Rice |
| Sn | *Senecio vulgaris* |
| Ip | *Ipomoea purpurea* |
| Am | *Amaranthus retroflexus* |
| Pi | *Polygonum aviculare* |
| Ca | *Chenopodium album* |
| Ga | *Galium aparine* |
| Xa | *Xanthium spinosum* |
| Ab | *Abutilon theophrasti* |
| Co | *Cassia obtusifolia* |
| Av | *Avenua fatua* |
| Dg | *Digitaria sanguinalis* |
| Al | *Alopecurus myosuroides* |
| St | *Setaria viridis* |
| Ec | *Echinochloa crus-galli* |
| Sh | *Sorghum halepense* |
| Ag | *Agropyron repens* |
| Cn | *Cyperus rotundus* |

CLAIMS

1. Diphenyl ether compounds of the formula (I)

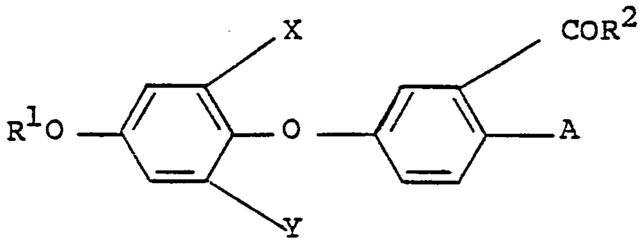

(I)

wherein $R^1$ is a haloalkyl group of 1 or 2 carbon atoms containing one or more fluorine or chlorine atoms;
X and Y are each independently hydrogen, halogen, $C_1-C_4$ alkyl, cyano, nitro or trifluoromethyl;
A is hydrogen, nitro, halogen, cyano, $CH_3$ or trifluoromethyl; and $R^2$ is (a) -OH, (b) -OM wherein M is a cation, (c) $-OR^3$ wherein $R^3$ is a phenyl group, a $C_1-C_6$ alkyl group, or a $C_2-C_6$ alkenyl or alkynyl group, each of which may optionally be substituted with halogen, trifluoromethyl, cyano, carboxy, $-CO_2M$, alkoxycarbonyl of 2 to 5 carbon atoms, amino, mono- or di-alkyl amino, wherein the alkyl groups have 1 to 6 carbon atoms, alkoxy of 1 to 6 carbon atoms, or phenyl, (d) $NR^4R^5$ wherein each of the groups $R^4$ and $R^5$ may be hydrogen or $C_1-C_6$ alkyl, $C_2-C_6$ alkenyl or alkynyl, or phenyl, each of which may optionally be substituted with any of the substituents recited above for the group $R^3$, or (e) $-NHSO_2R^3$, or $-NMSO_2R^3$ wherein M is a cation.

2. Compounds as claimed in claim 1 wherein the haloalkyl group $R^1$ is a $CF_3$, $ClCF_2$, $CF_2H$, $CH_3CF_2$ or $CHF_2CF_2$- group.

3. Compounds as claimed in claim 1 wherein $R^1$ is a $ClCF_2$ $CHF_2$- or $CF_3$ group; X is hydrogen, chlorine, or $NO_2$; Y is hydrogen, fluorine, or chlorine; $R^2$ is -OH, -OM, $-NH_2$, $-NHSO_2CH_3$, or $-NHSO_2CH_3$; and A is hydrogen, chlorine or $NO_2$.

4. A compound as claimed in claim 1 wherein $R^1$ is $CF_3$; X is chlorine; Y is hydrogen; $R^2$ is $-NHSO_2CH_3$ or $-NMSO_2CH_3$; and A is $NO_2$.

5. A compound as claimed in claim 1 wherein $R^1$ is $CF_3$; X is chlorine; Y is fluorine; $R^2$ is $-NHSO_2CH_3$ or $-NMSO_2CH_3$; and A is $NO_2$.

6. A herbicidal composition, comprising a compound of formula (I) as defined in any of claims 1 to 5, in admixture with a carrier comprising a solid or liquid diluent, and optionally further comprising a surface-active agent.

7. A herbicidal composition comprising a compound of the formula (I) as defined in any of claims 1 to 5, in admixture with another herbicide.

8. A method of inhibiting the growth of unwanted plants, which comprises applying to the plants, or to the locus thereof, a phytotoxic amount of a compound of formula (I) as defined in any of claim 1 to 5.

9. A process of preparing a compound of the formula (I) defined in claim 1, which comprises reacting together in the presence of a base either

(a) a phenol of formula 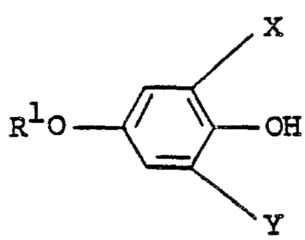 with a 5-halogenobenzoic acid derivative of the formula:

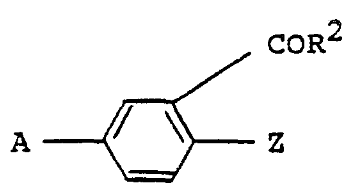

or

0063873

(b) a halogenophenyl ether of the formula 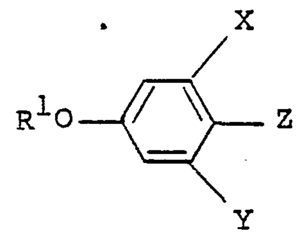

with a m-hydroxybenzoic acid derivative of the formula

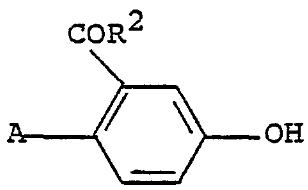

wherein $R^1$, $R^2$, A, X and Y have the meanings assigned to them in claim 1 and Z stands for fluorine or chlorine, and recovering the compound of formula (I).

10. A process of preparing a compound of formula (I) as defined in claim 1 which comprises reacting a suitably substituted hydroquinone with a 5-halogenobenzoic acid derivative of the formula:-

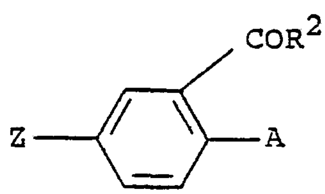

in presence of a base to obtain a compound of the formula

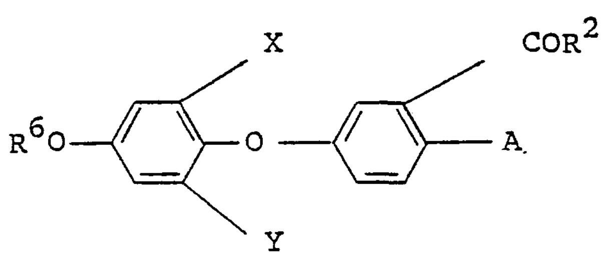

the symbols $R^2$, X, Y and A having the meaning assigned to them in claim 1, and Z being fluorine or chlorine, removing the protecting group $R^6$ if necessary and reacting the phenol compound with a haloalkyl chloride

0063873

$R^1Cl$ in the presence of a base, a solvent comprising a mixture of water and an organic solvent, and a phase-transfer catalyst, and recovering the compound of formula (I).

BAD ORIGINAL

1. Herbicidal compositions, comprising as an active ingredient a diphenyl ether compound of the formula (I)

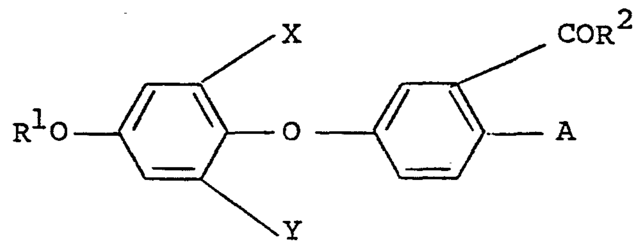

(I)

wherein $R^1$ is a haloalkyl group of 1 or 2 carbon atoms containing one or more fluorine or chlorine atoms;
X and Y are each independently hydrogen, halogen, $C_1$-$C_4$ alkyl, cyano, nitro or trifluoromethyl;
A is hydrogen, nitro, halogen, cyano, $CH_3$ or trifluoromethyl and $R^2$ is (a) -OH, (b) -OM wherein M is a cation, (c) $-OR^3$ wherein $R^3$ is a phenyl group, a $C_1$-$C_6$ alkyl group, or a $C_2$-$C_6$ alkenyl or alkynyl group, each of which may optionally be substituted with halogen, trifluoromethyl, cyano, carboxy, $-CO_2M$, alkoxycarbonyl of 2 to 5 carbon atoms, amino, mono- or di-alkyl amino, wherein the alkyl groups have 1 to 6 carbon atoms, alkoxy of 1 to 6 carbon atoms, or phenyl, (d) $NR^4R^5$ wherein each of the groups $R^4$ and $R^5$ may be hydrogen or $C_1$-$C_6$ alkyl, $C_2$-$C_6$ alkenyl or alkynyl, or phenyl, each of which may optionally be substituted with any of the substituents recited above for the group $R^3$, or (e) $-NHSO_2R^3$, or $-NMSO_2R^3$ wherein M is a cation; in admixture with a carrier comprising a solid or liquid diluent, and further optionally comprising a surface-active agent.

2. Herbicidal compositions as claimed in claim 1 wherein the haloalkyl group $R^1$ is a $CF_3$, $ClCF_2$, $CF_2H$, $CH_3CF_2$ or $CHF_2CF_2$- group.

3. Herbicidal compositions as claimed in claim 1 wherein $R^1$ is a $ClCF_2$, $CHF_2$ or $CF_3$ group; X is hydrogen, chlorine, or $NO_2$; Y is hydrogen, fluorine, or chlorine; $R^2$ is -OH, -OM, $-NH_2$, $-NHSO_2CH_3$, or $-NHSO_2CH_3$; and A is hydrogen, chlorine or $NO_2$.

4. A herbicidal composition as claimed in claim 1 wherein $R^1$ is $CF_3$; X is chlorine; Y is hydrogen; $R^2$ is $-NHSO_2CH_3$ or $-NMSO_2CH_3$; and A is $NO_2$.

5. A herbicidal composition as claimed in claim 1 wherein $R^1$ is $CF_3$; X is chlorine; Y is fluorine; $R^2$ is $-NHSO_2CH_3$ or $-NMSO_2CH_3$; and A is $NO_2$.

6. A herbicidal composition comprising a compound of the formula (I) as defined in any of claims 1 to 5, in admixture with another herbicide.

7. A method of inhibiting the growth of unwanted plants, which comprises applying to the plants, or to the locus thereof, a phytotoxic amount of a compound of formula (I) as defined in any of claims 1 to 5.

8. A process of preparing a compound of the formula (I) defined in claim 1, which comprises reacting together in the presence of a base either

(a) a phenol of formula $R^1O$ 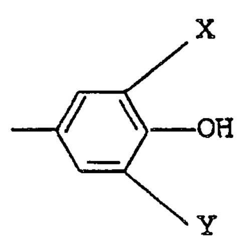 with a 5-halo-

genobenzoic acid derivative of the formula:

BAD ORIGINAL

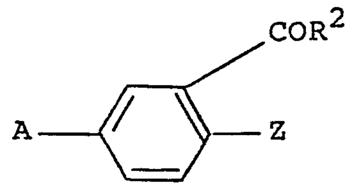

or

(b) a halogenophenyl ether of the formula 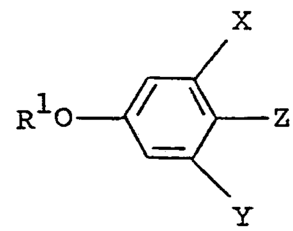

with a m-hydroxybenzoic acid derivative of the formula

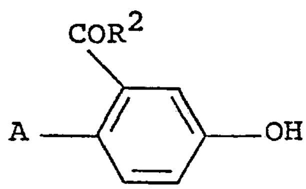

wherein $R^1$, $R^2$, A, X and Y have the meanings assigned to them in claim 1 and Z stands for fluorine or chlorine, and recovering the compound of formula (I).

9. A process of preparing a compound of formula (I) as defined in claim 1 which comprises reacting a suitably substituted hydroquinone with a 5-halogenobenzoic acid derivative of the formula:-

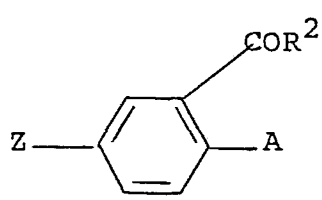

in presence of a base to obtain a compound of the formula

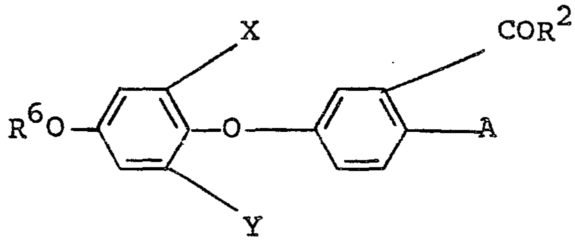

the symbols $R^2$, X, Y and A having the meaning assigned to them in claim 1, Z being fluorine or chlorine, and $R^6$ being hydrogen or a protecting group if either of X or Y is not hydrogen; removing the protecting group if present; and reacting the phenol compound with a haloalkyl chloride $R^1Cl$ in the presence of a base, a solvent comprising a mixture of water and an organic solvent, and a phase-transfer catalyst, and recovering the compound of formula (I).

European Patent Office

# EUROPEAN SEARCH REPORT

0063873

Application number

EP 82 30 1757

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 022 610 (MOBIL OIL CORP.) *Claims 1,3,4,7-9; page 13, lines 10-20* --- | 1-3,6, 8 | C 07 C 65/24<br>C 07 C 79/46<br>C 07 C 51/367<br>C 07 C 143/74<br>C 07 C 103/26<br>A 01 N 37/40<br>A 01 N 37/48<br>A 01 N 41/06 |
| A | US-A-4 209 318 (JOHNSON) *Claim 1; column 3, lines 32-41,54-59; column 5, lines 6-24,53-68; column 9, line 9 - column 10, line 16* --- | 1,6-9 | |
| A | EP-A-0 033 629 (MOBIL OIL CORP.) *Claim 1* ----- | | |
| | | | TECHNICAL FIELDS SEARCHED (Int. Cl. 3) |
| | | | C 07 C 65/00<br>C 07 C 79/00 |

The present search report has been drawn up for all claims

| Place of search | Date of completion of the search | Examiner |
|---|---|---|
| THE HAGUE | 28-06-1982 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document